(19) Europäisches Patentamt

European Patent Office

Office européen des brevets

(11) **EP 2 226 389 A1**

(12) **EUROPEAN PATENT APPLICATION**

(43) Date of publication:
**08.09.2010 Bulletin 2010/36**

(51) Int Cl.:
*C12Q 1/18* (2006.01)

(21) Application number: **09154142.5**

(22) Date of filing: **02.03.2009**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(71) Applicant: **DSM IP Assets B.V.
6411 TE Heerlen (NL)**

(72) Inventor: **Stark, Jacobus
3069 ZM Rotterdam (NL)**

(74) Representative: **Biermann, Jan et al
DSM Intellectual Property
Delft Office (600-0240)
P.O. Box 1
2600 MA Delft (NL)**

(54) **Test for determining a disinfectant concentration in a fluid**

(57) The present invention relates to a method for determining the concentration of a disinfectant in a fluid by means of a microbial inhibition assay. Furthermore, the present invention provides a method to optimize the concentration of a disinfectant in a fluid.

**EP 2 226 389 A1**

**Description**

**Field of the invention**

The present invention relates to a simple and easy-to-use method for the rapid determination of the concentration of a disinfectant in a fluid such as *e.g.* water.

**Background of the invention**

Many species of microorganisms such as bacterial species can survive and multiply in water. Growth of microorganisms in *e.g.* process, cooling, agricultural or drinking water may give too high microbial counts or may lead to the formation of biofilms in *e.g.* pipes and equipment. Furthermore, pathogenic microorganisms and/or toxic compounds produced by such microorganisms can contaminate recreational and domestic water supplies through the discharge of poorly treated industrial, agricultural and municipal wastewater. Despite large advances made in waste water treatment, the presence of pathogenic microorganisms in water supplies still poses a considerable world-wide health risk and is a causative factor in outbreaks of diseases including dysentery, cholera, typhoid and gastroenteritis. Waterborne pathogenic microorganisms infect around 250 million people each year resulting in 10 to 20 million deaths. Many of these infections occur in developing nations, however, numerous incidences with waterborne pathogenic microorganism related diseases have been reported in industrialized countries suggesting that the general community is still at risk from waterborne diseases caused by pathogenic microorganisms.

To reduce the microbial counts in *e.g.* process or cooling water and to minimize the chances of too high amounts of harmful microorganisms occurring in water, whether for purposes of recycling or reuse in industrial processes or prior to its discharge into the environment, treatment with disinfectants is often applied.

When disinfectants are used, dosage control is of importance. On the one hand, the amount of disinfectant should not be too low, as this might result in survival and growth of unwanted microorganisms in the water which can lead to economic losses and/or health hazards. On the other hand, the amount of disinfectant should not be too high, as overdosing is unattractive from an economical point of view and further might cause excessive, and therefore polluting, quantities of disinfectants or its toxic byproducts, a number of which are mutagenic and/or carcinogenic, to get into the environment.

The amount of disinfectants in water can be measured by means of several methods including photometry, electrochemistry, gas chromatography and direct liquid chromatography (see Pettas and Karayannis, 2004). These techniques are however laborious and time-consuming and often need expensive devices and well trained personnel.

Thus, a need exists for a simple, inexpensive and easy-to-use method for determining the concentration of a disinfectant in a fluid such as *e.g.* water. The present invention provides such a method.

**Summary of the invention**

It is an object of the present invention to provide a simple, inexpensive and easy-to-use method for determining the concentration of a disinfectant in a fluid such as *e.g.* water. Also provided is a method to optimize the concentration of a disinfectant in a fluid. Surprisingly, it has been found that such methods can be provided when a microbiological test method is applied. The microbiological test method includes a ready-to-use test system that makes use of a microorganism and gives a result by the change indicated by an indicator molecule added to the test system. The principle is that when disinfectant is present in the fluid in a concentration sufficient to inhibit the growth of the microorganism the color of the indicator will stay the same, while, when no inhibition occurs, the growth of the microorganism is accompanied by the formation of acid and/or reduced metabolites or other phenomena that will induce an indicator signal.

**Detailed description of the invention**

In a first aspect the invention pertains to a method for determining the concentration of a disinfectant in a fluid, the method comprising the steps of:

  (a) taking a sample from the fluid,
  (b) preparing a dilution range of the sample,
  (c) contacting each dilution obtained in step (b) with a test medium comprising a microorganism, a nutrient and an indicator,
  (d) incubating each mixture obtained in step (c) for a period of time to grow the microorganism in case no disinfectant is present in the sample,
  (e) detecting growth or inhibition of growth of the microorganism with the indicator,

(f) determining the dilution with the highest dilution factor capable of inhibiting the growth of the microorganism,

(g) determining the concentration of the disinfectant in the dilution determined in step (f), and

(h) determining the concentration of the disinfectant in the fluid by multiplying the concentration determined in step (g) with the dilution factor used to make the dilution.

"Disinfectant" as used herein means antimicrobial agents used to destroy microorganisms. Disinfectants reduce the number of microorganisms or even might eliminate some or all types of microorganisms. Examples of disinfectants are sanitizers, antiseptics and antibiotics. They include, but are not limited to, alcohols such as ethanol or isopropanol; aldehydes such as ortho-phthalaldehyde or glutaraldehyde; oxidizing agents such as liquid gas chlorine, chlorine dioxide, hypochlorite or chloramine compounds, hydrogen peroxide, ozone, iodine, peracetic acid, performic acid, potassium permanganate, potassium peroxymonosulfate; phenolic compounds such as phenol, thymol, O-phenylphenol, chloroxylenol; quaternary ammonium compounds such as benzalkonium chloride; antimicrobials such as *e.g.* beta-lactams, tetracyclines, aminoglycosides, quinolones and sulfonamides and derivatives thereof; and any combination thereof.

The fluid can be waste water; process water; sewage water; drinking water; water applied in *e.g.* swimming pools, saunas and greenhouses; and cooling water. It can be water from agricultural sources; fisheries; ship ballast; cooling towers; waste water treatment plants; power plants; chemical industries such as textile industry, paper and pulp industry, printing industry, iron-steel industry, coke industry, petroleum industry, pesticide industry, paint industry, medical and dental industry, solvent industry, pharmaceutical industry, wood preserving chemicals industry, and food industry. The fluid can be an incoming effluent and/or stream, an outgoing effluent and/or stream, or an intermediate effluent and/or stream from any of the above sources.

In a first step a sample is taken from the fluid. This can be done manually, semi-automatically or automatically. One or several samples can be taken. The sample can be taken from the fluid by means of a sampling device. The term "sampling device" refers to a device with the aid of which a sample of a fluid can be taken and optionally added to dilution medium. Of course, the dilution medium may also be added to the sample. A sampling device may be a container, optionally with volume markings. Such a container may be a capillary, a syringe, a pipette or an automated pipetting system. A syringe or pipette may be designed in such a way that with only one mode of operation a predetermined volume can be withdrawn from the fluid to be analyzed.

In a second step a dilution range of the sample (or the samples) is prepared. This again can be done manually, semi-automatically or automatically. If several samples are taken, several dilution ranges can be prepared. The dilution range can be prepared by making as many dilutions as desired by diluting the sample with a suitable dilution medium (*e.g.* water). A sampling device as defined above can be used to make the dilution range.

In a third step each dilution made in the second step is contacted with a test system. The test system comprises a test medium comprising a microorganism, a nutrient and an indicator.

"Microorganism" as used herein refers to a microorganism that is sensitive towards the disinfectant and which is suitable for monitoring the presence or absence of the disinfectant in a sample by means of its growth. The term "sensitivity" as used herein refers to the degree by which concentrations of disinfectants in a sample can be determined by the test system. A suitable test system should have the desired sensitivity with regard to the compounds to be tested for. In an embodiment the microorganism is a strain of *Bacillus, Escherichia coli* or *Streptococcus*. In a further embodiment the microorganism is a thermo stable microorganism such as *Bacillus stearothermophilus* or *Streptococcus thermophilus*. The test medium may comprise one or more types of microorganisms as detecting agents. The microorganism may be introduced in the test medium as units capable of producing colonies, or CFUs. The term "CFU" is an abbreviation of Colony Forming Units and refers to the number of microorganisms, spores of microorganisms, partially germinated spores of microorganisms and/or vegetative cells capable of producing colonies of microorganisms. The term "spore" as used herein refers to a primitive usually unicellular often environmentally resistant dormant or reproductive body produced by microorganisms and capable of development into a new individual microorganism. The concentration of said CFU's is expressed as Colony Forming Units per ml of test medium (CFU/ml) and is usually in the range of $1 \times 10^5$ to $1 \times 10^{12}$ CFU/ml, preferably $1 \times 10^6$ to $1 \times 10^{10}$ CFU/ml, more preferably $2 \times 10^6$ to $1 \times 10^9$ CFU/ml, most preferably $5 \times 10^6$ to $1 \times 10^8$ CFU/ml, or still more preferably $5 \times 10^6$ to $2 \times 10^7$ CFU/ml.

The term "nutrient" as used herein refers to a nutritive substance or ingredient that promotes and/or is required for the growth of the microorganism. Suitable nutrients depend from the microorganism used in the test system. The test medium may comprise two or more different nutrients. They include, but are not limited to, assimilable carbon sources such as carbohydrates such as *e.g.* glucose, fructose, sucrose, lactose and dextrose; assimilable nitrogen sources such as amino acids such as *e.g.* peptone or tryptone; sources of vitamins and growth factors such as beef or yeast extract; and sources of minerals such as earth alkaline metal salts such as salts of *e.g.* barium or calcium.

The term "indicator" refers to a substance used to measure (for example by change of colour or fluorescence) the condition of a test medium with respect to the presence of a particular component (for example an acid, a base, oxidizing or reducing agents). The indicator, upon changing from one state to another, provides a detectable signal such as a change in color or fluorescence. The indicator may be a pH-indicator, a redox-indicator or a combination thereof.

The term also may refer to two or more indicators. Examples of suitable indicators are well known to the skilled artisan (see handbook H.J. Conn's Biological Stains, R.D. Lillie ed., Baltimore, 1969).

The test medium may have the form of a liquid, a solid or a gel-like matrix. When the test medium has the form of a gel-like matrix, it may comprise a gelling agent. Suitable examples of gelling agents are agar, alginic acid and salts thereof, carrageenan, gelatin, hydroxypropylguar and derivatives thereof, locust bean gum (Carob gum), processed eucheuma seaweed and the like. In an embodiment the microorganism, the indicator and the nutrient are introduced into an agar solution. The agar solution is allowed to solidify to form the test medium such that the microorganism stays alive, but cannot multiply because of *e.g.* low temperature. When the test medium has the form of solid matrix, it may comprise a carrier material such as a ceramic, cotton, glass, a metal particle, a polymer in any shape or form, a silicate, a sponge, wool and the like. Alternatively, the test medium may have the form of a tablet, disc or paper filter comprising the microorganism, indicator and nutrient. The three constituents may be present in a single tablet, but also in two or more tablets. Of course, test systems combining test media in solid, liquid and/or gel-like form may be used. In an embodiment the amount of gelling agent in the test medium is between 2 and 100 g/l, preferably between 5 and 50 g/l, more preferably between 10 and 20 g/l, most preferably between 12 and 15 g/l. Ina preferred embodiment the gelling agent is agar.

Optionally, the test medium may also contain one or more buffers; stabilizers; surfactant; salts; substances that change the sensitivity to certain antimicrobial compounds in a positive or negative way; viscosity-increasing agents; or any combination thereof. When a buffer is present in the medium, it may be added during the mixing of the components of the medium or the components may be dissolved and/or suspended in the buffer. Suitable buffering agents include, but are not limited to, alanine, potassium phosphate and sodium phosphate. Examples of substances that change the sensitivity to certain antimicrobial compounds are antifolates like ormethoprim, tetroxoprim and trimethoprim that improve the sensitivity of the microorganism towards sulfa compounds or salts of oxalic acid or hydrofluoric acid or calcium chelating agents which improve the sensitivity towards tetracycline. Cysteine and penicillin binding protein are compounds that are known to decrease the sensitivity to certain antimicrobial compounds. Examples of viscosity-increasing agents include, but are not limited to, ascorbyl methylsilanol pectinate, carbomer, carboxymethyl cellulose, cetearyl alcohol, cetyl alcohol, cetyl esters, cocamide DEA, emulsifying wax, glucose, hydroxyethyl cellulose, hydroxypropylmethyl cellulose, lauramide DEA, linoleamide DEA, magnesium aluminum silicate, maltodextrins, PEG-8 distearate, polyacrylamide, polyvinyl alcohol, PVP/hexadecene copolymer, sodium chloride, sodium sulfate, soyamidopropyl betaine, xanthan gum and the like. A suitable stabilizer is *e.g.* colloidal silica. Alternatively, the optional ingredients of the test medium mentioned above may also be added exogenously.

The test medium may be contained within any type of container; frequently used containers are tubes, microtiter plates and petri dishes. The containers may be of any shape and size and from any material available, provided that observation of indicator changes is possible. Optionally, means for sealing of said containers filled with test medium during incubation and/or an insert with instructions for use and/or a means for setting the time needed for incubation are part of the test system.

Optionally, the test medium is sterilized and usually the pH is adjusted to the required value. The method of the present invention includes mixing samples (*e.g.* with other samples, but also with *e.g.* salts, buffering compounds, stabilizers, isotope-labeled compounds, fluorescence-labeled compounds and the like), concentrating and/or further diluting (*e.g.* with diluting liquids such as water) samples prior to addition to the test medium.

In a fourth step each mixture obtained in the third step is incubated for a period of time to grow the microorganism in case no disinfectant is present in the sample. In other words, after addition of the sample, growth of the microorganism is allowed to take place during a period sufficiently long for the microorganisms to grow in case no antibiotics are present by *e.g.* raising the temperature and/or providing for a pH-value at which the microorganism is able to grow. The growth of the microorganism is to take place during a predetermined period, preferably within a time span of 0.5 to 4 hours, more preferably between 1 to 3.5 hours, most preferably between 2.0 to 3.25 hours. Preferably the growth of the microorganism is conducted at a predetermined temperature, preferably the optimal growth temperature of the microorganism. When, for example, thermo stable microorganisms are used, said temperature preferably is between 40 and 70°C, more preferably between 50 and 65°C, most preferably between 60 and 64°C. Optionally, said reaction can be carried out with the aid of a thermostatic device. With the aid of the thermostatic device test samples can be kept at a pre-set temperature, such as the temperature at which the microorganism shows sufficient growth. Preferably, said thermostatic device is designed in such a fashion that it can hold containers filled with test medium. Optionally, the thermostatic device is coupled to a means for setting the time needed for incubation such that heating and/or cooling is stopped after lapse of a pre-set period. Alternatively, the time required for growth of the microorganism is equal to the time that is required for a calibration sample without any disinfectant to induce a change in the indicator.

In a fifth step growth or inhibition of growth of the microorganism is detected with the indicator. In other words, growth or inhibition of growth of the microorganism is detected by observing the presence or absence of a change of the indicator. Preferably, this is done for each dilution of the dilution range. When, for example the change of the indicator is a color change, the color change may be observed visually. However, in an embodiment of the invention the color change is determined using an arrangement that generates digital image data or an arrangement that generates analog image data and converts said analog image data into digital image data followed by interpretation of said digital image data by a computer processor. Such an arrangement, which may for instance be a sample-reading device such as a scanner coupled to a personal computer, is described in WO 03/033728, which is hereby incorporated by reference, and briefly summarized hereafter. With this arrangement it is possible to scan the bottom side of each of the samples in a test plate. The color and the brightness of the reflected light are registered in three variables, each describing one color component, for instance the so-called L*a*b* model. In the L*a*b* model, the color spectrum is divided in a two-dimensional matrix. The position of a color in this matrix is registered by means of the two variables "a" and "b". The variable L indicates the intensity (for instance, from light blue to dark-blue). It is possible to make a criterion comprising the a-value, b-value and L-value to make a composite function as follows:

$$Z = w_L.L + w_a.a + w_b.b$$

, wherein $W_L$, $W_a$ and $W_b$ are weighting factors for the L-value, a-value and b-value, respectively. The values of these weighting factors can be calculated by means of "discriminent analysis", such that the group means show a maximum distance in relation to the spreading. By combining two or more of the color components in the L*a*b* model in a predetermined manner that depends on the type of disinfectant and the sample, an accurate detection is possible. In practice, a certain value of Z at which a test should switch between positive and negative result is experimentally predetermined for each disinfectant. If desired, reading and computer equipment described above can be combined with heating equipment *e.g.* a thermostatic device as described in WO 2007/090683, which is also incorporated by reference herein.

The test system may also comprise a data carrier loaded with a computer program suitable for instructing a computer to analyze digital data obtained from a sample-reading device. Said data carrier may be any carrier suitable for storing digital information such as a CD-ROM, a diskette, a DVD, a memory stick, a magnetic tape or the like. Advantageously, the data carrier loaded with a computer program provides for easy access to the latest available computer programs suitable for use in the method of the present invention.

In a sixth step the dilution with the highest dilution factor (*i.e.* the dilution with the lowest concentration disinfectant) capable of inhibiting the growth of the microorganism is determined. In other words, the dilution of the dilution range prepared in the second step that is capable of inhibiting the growth of the microorganism as measured by means of the above described test system is determined.

In a seventh step the concentration of the disinfectant in the dilution that is determined in the sixth step is determined. The concentration can be determined with any method suitable for determining the concentration of the respective disinfectant in the dilution. In a preferred embodiment the seventh step comprises the steps of:

(i) preparing samples, each sample having a different concentration of the disinfectant; the concentration of the disinfectant in each sample is known.
(ii) contacting each sample obtained in step (i) with a test medium comprising a microorganism, a nutrient and an indicator,
(iii) incubating each mixture obtained in step (ii) for a period of time to grow the microorganism in case no disinfectant is present in the sample,
(iv) detecting growth or inhibition of growth of the microorganism with the indicator,
(v) determining the sample with the lowest disinfectant concentration capable of inhibiting the growth of the microorganism,
(vi) determining the concentration of the disinfectant in the dilution by correlating it to the concentration of the sample determined in step (v).

Various aspects concerning the test systems and test media and its constituents are the same as has been outlined above. In the preferred embodiment of the seventh step a calibration range (comprising various samples, each sample having a different known concentration of the disinfectant) is prepared and the transition point between growth and no growth of the microorganism for a specific disinfectant or combination of disinfectants within the pre-set time and temperature is determined. In this step the lowest concentration of a specific disinfectant or combination of disinfectants at which the test does not switch color can be determined. The obtained concentration can then be correlated to the concentration of the disinfectant in the dilution. In an embodiment the concentrations relate to one another and might be similar or even identical.

In the eight step the concentration of the disinfectant in the fluid is determined by multiplying the concentration determined in the seventh step with the dilution factor used to make the dilution.

In a further aspect the invention relates to a method to optimize the concentration of a disinfectant in a fluid, the method comprising the steps of:

(a) performing the method for determining the concentration of a disinfectant in a fluid according to the present invention,
(b) decreasing or increasing the concentration of the disinfectant in the fluid to a desired value,
(c) optionally, repeating step (a), and
(d) optionally, repeating step (b) until the desired value has been reached.

In an embodiment the desired concentration value is higher than the lowest disinfectant concentration capable of inhibiting the growth of the microorganism.

Most spoilage organisms and/or organisms which can form biofilms in *e.g.* equipment, storage tanks or pipelines cam be classified as bacteria. Most pathogenic organisms can be classified as protozoan, bacteria or viruses. The viruses of particular concern to the treatment of waste water, process water, sewage water or cooling water include, but are not limited to, water-borne polio viruses, including rotaviruses. Bacteria comprise the largest group of pathogenic microorganisms of particular concern to the treatment of waste water, process water, sewage water or cooling water and include, but are not limited to, *Salmonella* sp., *Shigella* sp., *Escherichia coli*, and a broad variety of others generally known. The most common bacteriological diseases include shigellosis, which causes dysentery, food poisoning, and cholera. Protozoan pathogens of particular concern to the treatment of waste water, process water, sewage water or cooling water include *Giardia* sp. which causes giardiasis, one of the most prevalent water-borne diseases in the United States, and *Cryptosporidium* sp., which causes dysentery.

The amount of disinfectant added to *e.g.* processing water or cooling is referred to as the "dosage" and is usually expressed as milligrams per liter (mg/l) or parts per million (ppm). The amount of disinfectant necessary to disinfect a particular volume of water is referred to as the "demand". The reaction between the disinfectant and the contaminants is typically not instantaneous but is instead time dependent. In order to obtain adequate disinfection, the mixing of water and disinfectant should be completed in the shortest time possible, ideally a fraction of a second. The amount of disinfectant remaining in the water at the time of measurement is referred to as the "residual." The residual is therefore determined by the demand subtracted from the dosage. Generally, the water of interest comprise disinfectant in a concentration that is sufficient to kill the pathogenic organisms of concern (*i.e.* the demand or desired value). If the concentration of the disinfectant in the fluid differs from the desired value, it can be optimized by means of the above method for it to become the desired value again. If the concentration of the disinfectant in the fluid is higher than the desired value, the dosage can be adjusted (*i.e.* decreased) or the waste water, process water, sewage water and/or cooling water can be diluted. If the concentration of the disinfectant in the fluid is lower than the desired value, the dosage can also be adjusted (*i.e.* increased).

In an embodiment the ratio of the dilution/sample to test medium exceeds 2:3 (0.68:1) (v/v). Preferably, said ratio is at least 20:27 (0.74:1) (v/v), more preferably said ratio is at least 25:27 (0.93:1) (v/v); most preferably said ratio is at least 2:1 (v/v). It has been found that there is no technical reason for an upper limit to the amount of dilution/sample. In practice this volume should not exceed the maximum content of the container that holds the test medium. For example, in a 2 ml container having 0.2 ml test medium, no more than 1.8 ml of fluid sample should be added. In practice, containers for performing the method of the present invention have a volume that rarely exceeds 50 ml and hence the amount of fluid sample to be added shall not exceed 50 ml, preferably 10 ml, more preferably 5 ml, still more preferably 2 ml, most preferably 1 ml. Thus, in general, the upper limit of the ratio of the volume of fluid sample to the volume of test medium is 250:1 (v/v), preferably 50:1 (v/v), more preferably 25:1 (v/v), still more preferably 10:1 (v/v), most preferably 5:1 (v/v). In a preferred embodiment the volume of the dilution/sample is greater than the volume of test medium.

**REFERENCES**

Pettas and Karayannis (2004), Simultaneous spectra-kinetic determination of peracetic acid and hydrogen peroxide in a brewery cleaning-in-place disinfection process. Anal.Chim. Acta 522:275-280

**EXAMPLES**

**Example 1**

**Determining the concentration of a disinfectant in waste water**

A process water stream is treated with a disinfectant to kill spoilage and/or biofilm generating bacteria. The desired concentration value of the disinfectant in the process water is 220 ppm. This concentration is necessary to keep the bacteria in the process water at a low and acceptable number. To determine if the concentration of the disinfectant in the process water stream is still at the desired value (the demand), a sample is taken from the process water stream and a dilution range of the sample is prepared. The sample is serially diluted ten times by a dilution factor 2 with water. So, the final dilution range comprises eleven dilutions: dilution 1 comprising disinfectant concentration X; dilution 2 comprising disinfectant concentration 1/2 X; dilution 3 comprising disinfectant concentration 1/4X; dilution 4 comprising disinfectant concentration 1/8X; dilution 5 comprising disinfectant concentration 1/16X; dilution 6 comprising disinfectant concentration 1/32X; dilution 7 comprising disinfectant concentration 1/64X; dilution 8 comprising disinfectant concentration 1/128X; dilution 9 comprising disinfectant concentration 1/256X; dilution 10 comprising disinfectant concentration 1/512X; dilution 11 comprising disinfectant concentration 1/1024X. As a control, a sample comprising only water is included.

Hundred microliter of each dilution is contacted with a Delvotest® test ampoule comprising a test medium comprising a thermo stable *Bacillus stearothermophilus* strain, nutrients and a pH-indicator and incubated in an incubator at about 64°C for about 3 hours. The color of the indicator in the test medium before the assay is performed is purple. When the microorganism in the test medium grows the test becomes yellow and when no growth is observed the test medium stays purple.

The results are shown in Table 1. The results demonstrate that a color change is observed in the control sample as well as dilutions 7 to 11, meaning that in these samples the microorganism present in the test medium grows. In dilutions 1 to 6 no color change is observed meaning that no growth of the microorganism is observed. Dilution 6 is the dilution with the highest dilution factor capable of inhibiting the growth of the microorganism.

Table 1. Microbial inhibition test performed on dilution range.

| Dilution | Color observed in microbial inhibition test |
|---|---|
| Control | Yellow |
| Dilution 1 | Purple |
| Dilution 2 | Purple |
| Dilution 3 | Purple |
| Dilution 4 | Purple |
| Dilution 5 | Purple |
| Dilution 6 | Purple |
| Dilution 7 | Yellow |
| Dilution 8 | Yellow |
| Dilution 9 | Yellow |
| Dilution 10 | Yellow |
| Dilution 11 | Yellow |

Next, a calibration range of the disinfectant is made in water. Ten samples each comprising a different concentration of the disinfectant are prepared. Sample 1 comprises 2 ppm disinfectant; Sample 2 comprises 4 ppm disinfectant; Sample 3 comprises 6 ppm disinfectant; Sample 4 comprises 8 ppm disinfectant; Sample 5 comprises 10 ppm disinfectant; Sample 6 comprises 12 ppm disinfectant; Sample 7 comprises 14 ppm disinfectant; Sample 8 comprises 16 ppm disinfectant; Sample 9 comprises 18 ppm disinfectant; Sample 10 comprises 20 ppm disinfectant. As a control, a sample comprising only water is included.

Hundred microliter of each sample is contacted with a Delvotest® test ampoule comprising a test medium comprising a thermo stable *Bacillus stearothermophilus* strain, nutrients and a pH-indicator and incubated in an incubator at about 64°C for about 3 hours. The color of the indicator in the test medium before the assay is performed is purple. When the microorganism in the test medium grows the test becomes yellow and when no growth is observed the test medium stays purple.

The results are shown in Table 2. Sample 3 is the sample with the lowest disinfectant concentration capable of inhibiting the growth of the microorganism.

Table 2. Microbial inhibition test performed on calibration range.

| Sample | Color observed in microbial inhibition test |
|---|---|
| Control | Yellow |
| Sample 1 | Yellow |
| Sample 2 | Yellow |
| Sample 3 | Purple |
| Sample 4 | Purple |
| Sample 5 | Purple |
| Sample 6 | Purple |
| Sample 7 | Purple |
| Sample 8 | Purple |
| Sample 9 | Purple |
| Sample 10 | Purple |

In a next step, the lowest disinfectant concentration capable of inhibiting the growth of the microorganism is correlated to the dilution with the highest dilution factor capable of inhibiting the growth of the microorganism. The former is sample 3 and the latter is dilution 6. Sample 3 comprises 6 ppm disinfectant. From the above can be concluded that dilution 6 has a disinfectant concentration of 6 ppm.

Dilution 1 has been diluted 32 times to become dilution 6. Therefore, the dilution factor is 32. The concentration of the disinfectant in the fluid is 192 ppm (=6 ppm x 32). As indicated above, the desired concentration of the disinfectant to be able to keep the number of bacteria in the process water at a low and acceptable number (without leaving to much residual disinfectant in the process water) is 220 ppm. Consequently, the concentration of disinfectant in the process water has to be increased such that the desired value is obtained.

**Claims**

1.  A method for determining the concentration of a disinfectant in a fluid, the method comprising the steps of:

> (a) taking a sample from the fluid,
> (b) preparing a dilution range of the sample,
> (c) contacting each dilution obtained in step (b) with a test medium comprising a microorganism, a nutrient and an indicator,
> (d) incubating each mixture obtained in step (c) for a period of time to grow the microorganism in case no disinfectant is present in the sample,
> (e) detecting growth or inhibition of growth of the microorganism with the indicator,
> (f) determining the dilution with the highest dilution factor capable of inhibiting the growth of the microorganism,
> (g) determining the concentration of the disinfectant in the dilution determined in step (f), and
> (h) determining the concentration of the disinfectant in the fluid by multiplying the concentration determined in step (g) with the dilution factor used to make the dilution.

2.  A method to optimize the concentration of a disinfectant in a fluid, the method comprising the steps of:

> (a) performing the method according to claim 1,
> (b) decreasing or increasing the concentration of the disinfectant in the fluid to a desired value,
> (c) optionally, repeating step (a), and
> (d) optionally, repeating step (b) until the desired value has been reached.

3.  A method according to claim 1 or 2, wherein step (g) of claim 1 comprises the steps of:

> (i) preparing samples, each sample having a different concentration of the disinfectant,
> (ii) contacting each sample obtained in step (i) with a test medium comprising a microorganism, a nutrient and an indicator,

(iii) incubating each mixture obtained in step (ii) for a period of time to grow the microorganism in case no disinfectant is present in the sample,

(iv) detecting growth or inhibition of growth of the microorganism with the indicator,

(v) determining the sample with the lowest disinfectant concentration capable of inhibiting the growth of the microorganism,

(vi) determining the concentration of the disinfectant in the dilution by correlating it to the concentration of the sample determined in step (v).

4. A method according to claim 2 or 3, wherein the desired concentration value is higher than the lowest disinfectant concentration capable of inhibiting the growth of the microorganism.

5. A method according to any of the claims 1 to 4, wherein the disinfectant is selected from the group consisting of alcohols, aldehydes, oxidizing agents, phenolic compounds, quaternary ammonium compounds, antimicrobials, and any combination thereof.

6. A method according to any of the claims 1 to 35 wherein the fluid is selected from the group consisting of waste water, process water, sewage water, drinking water, water applied in swimming pools, saunas or greenhouses, and cooling water.

7. A method according to any one of the claims 1 to 6, wherein the volume of the dilution or the sample is greater than the volume of the test medium.

Europäisches
Patentamt

European
Patent Office

Office européen
des brevets

## EUROPEAN SEARCH REPORT

Application Number

EP 09 15 4142

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 3 126 325 A (ELI LILLY) 24 March 1964 (1964-03-24) * column 1, line 18 - line 31 * * column 2, line 22 - line 39 * * column 2, line 68 * * examples 1-8 * * claims 1-4 * ----- | 1-7 | INV. C12Q1/18 |
| X | WO 2005/118837 A (DSM IP ASSETS BV [NL]; LANGEVELD PIETER CORNELIS [NL]; PELT VAN JOHANN) 15 December 2005 (2005-12-15) * page 9 * * claims 1-12 * ----- | 1-7 | |
| X | STEAD ET AL: "Evaluation and validation according to international standards of the Delvotest<(>R) SP-NT screening assay for antimicrobial drugs in milk" INTERNATIONAL DAIRY JOURNAL, ELSEVIER APPLIED SCIENCE, BARKING, GB, vol. 18, no. 1, 21 November 2007 (2007-11-21), pages 3-11, XP022355633 ISSN: 0958-6946 * the whole document * ----- | 1-7 | TECHNICAL FIELDS SEARCHED (IPC) C12Q |

The present search report has been drawn up for all claims

| Place of search | Date of completion of the search | Examiner |
|---|---|---|
| Munich | 26 March 2009 | Bayer, Martin |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

&  : member of the same patent family, corresponding
     document

EPO FORM 1503 03.82 (P04C01)

**EP 2 226 389 A1**

## ANNEX TO THE EUROPEAN SEARCH REPORT
## ON EUROPEAN PATENT APPLICATION NO.

EP 09 15 4142

This annex lists the patent family members relating to the patent documents cited in the above-mentioned European search report.
The members are as contained in the European Patent Office EDP file on
The European Patent Office is in no way liable for these particulars which are merely given for the purpose of information.

26-03-2009

| Patent document cited in search report | | Publication date | Patent family member(s) | | Publication date |
|---|---|---|---|---|---|
| US 3126325 | A | 24-03-1964 | NONE | | |
| WO 2005118837 | A | 15-12-2005 | CA | 2567102 A1 | 15-12-2005 |
| | | | US | 2008020420 A1 | 24-01-2008 |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 03033728 A

- WO 2007090683 A

**Non-patent literature cited in the description**

- handbook H.J. Conn's Biological Stains. 1969

- **Pettas ; Karayannis.** Simultaneous spectra-kinetic determination of peracetic acid and hydrogen peroxide in a brewery cleaning-in-place disinfection process. *Anal.Chim. Acta,* 2004, vol. 522, 275-280